# EUROPEAN PATENT APPLICATION

(11) **EP 4 014 871 A1**
(43) Date of publication of application: **22.06.2022**
(21) Application number: 21215489.2
(22) Date of filing: 17.12.2021
(51) Int. Cl.: A61B 5/367, A61B 5/00

(54) **AUTOMATIC POST PACING INTERVAL MEASUREMENT AND DISPLAY**

(30) Priority: 18.12.2020 US 202017127227
(71) Applicant: Biosense Webster (Israel) Ltd, Yokneam, 2066717 (IL)
(72) Inventor: BOTZER, Lior, 2066717 Yokneam (IL); GLINER, Vadim, 2066717 Yokneam (IL); GOVARI, Assaf, 2066717 Yokneam (IL)
(74) Representative: Carpmaels & Ransford LLP

(57) **Abstract**

A method is provided. The method is implemented by a determination engine executed stored on a memory as processor executable code that is executed by a processor. The method includes determining that a stimulating pacing signal is captured from at least a portion of an anatomical structure and determining a time duration between the stimulating pacing signal and a subsequent response from the portion of an anatomical structure. The method further includes outputting the time duration.

## Description

### FIELD OF INVENTION

The present invention is related to a method and system for signal processing. More particularly, the present invention relates to a method and system for signal processing that provides automatic post pacing interval measurement and display.

### BACKGROUND

Treatments for cardiac conditions, such as cardiac arrhythmia, often require analyzing a specific region or focal point of heart tissue via pacing by a catheter. Pacing is a technique used to identify a source (i.e., a focal point) of a cardiac arrhythmia by using electrodes of the catheter to provide a very short pulse of electrical current to activate tissue in a given region (e.g., a pacing/collection site) and to generate an electric wave from a particular point (e.g., a sinus rhythm from an excitable gap of a circuit). The given region may be large, similar to the area covered by a patch of a defibrillator. The sinus rhythm can be likened to dominos, as waves of the sinus rhythm follow one after the other.

An aspect of pacing includes identifying a post pacing interval (PPI). A PPI, generally, is a time from a last pace signal to a next automatic activity. In some cases, a PPI can be a time required for a last stimulated wave front to reach an excitable gap of a circuit, travel around the circuit, and return to a pacing/collection site (e.g., the given region). If the pacing site is in the circuit (e.g., the given region and the particular point coincide), then the PPI equates to tachycardia cycle length (TCL). Yet, as a pacing site is increasing distant from a circuit, a PPI-TCL difference corresponding increase.

Conventionally, cardiac physicians manually determine PPIs. For example, a cardiac physician provides a simulation pacing signal and manually determines a PPI (e.g., 200 milliseconds) for the simulation pacing signal to return to a pacing/collection site. Then, for that PPI, the cardiac physician inserts a clinical understanding. However, this conventional determination is time consuming and prone to inaccuracies. It would be advantageous to automatically observe and/or determine the PPIs. Therefore, a method for automatic PPI measurement and display is desirable.

### SUMMARY

According to an exemplary embodiment, a method is provided. The method is implemented by a determination engine executed stored on a memory as processor executable code that is executed by a processor. The method includes determining that a stimulating pacing signal is captured from at least a portion of an anatomical structure and determining a time duration between the stimulating pacing signal and a subsequent response from the portion of an anatomical structure. The method further includes outputting the time duration.

According to one or more embodiments, the exemplary method embodiment above can be implemented as an apparatus, a system, and/or a computer program product.

### BRIEF DESCRIPTION OF THE DRAWINGS

A more detailed understanding may be had from the following description, given by way of example in conjunction with the accompanying drawings, wherein like reference numerals in the figures indicate like elements, and wherein:
FIG. 1 illustrates a diagram of an exemplary system in which one or more features of the disclosure subject matter can be implemented according to one or more embodiments;
FIG. 2 illustrates a block diagram of an example system for automatic post pacing interval measurement and display according to one or more embodiments;
FIG. 3 illustrates an exemplary method according to one or more embodiments;
FIG. 4 illustrates an exemplary method according to one or more exemplary embodiments; and
FIG. 5 illustrates an exemplary interface according to one or more embodiments.

### DETAILED DESCRIPTION

Disclosed herein is a pacing and diagnosis method and system for signal processing, and the signal processing by pacing and diagnosis method and system relates to at least automatic PPI measurement and display. More particularly, the cardiac pacing and diagnosis apparatus includes a processor executable code or software that is necessarily rooted in process operations by, and in processing hardware of, medical device equipment to provide a method for automatic PPI measurement and display.

According to an embodiment, the cardiac pacing and diagnosis apparatus provides specific pacing and capturing operations that involve a multi-step manipulation of electrical signals with respect to tissue of an anatomical structure to understand an electrophysiology of that structure tissue with more precision. For ease of explanation, the pacing and diagnosis method and system is described herein with respect to mapping a heart; however, any anatomical structure, body part, organ, or portion thereof can be a target for mapping by the pacing and diagnosis method and system described herein.

For example, the pacing and diagnosis method and system includes a determination engine. That is, as the pacing and diagnosis method and system performs pacing techniques, the pacing and diagnosis method and system needs to know how long the pacing itself occurs (e.g., a timing of the pacing) and to identify when activity passes the electrodes 111 (e.g., measure a time until a next activity), as well as being sure that the pacing captures tissue (e.g., pacing of a focal point was successful). Since there is always a possibility that the tissue is not captured do to the catheter not being in correct contact, to pacing at a wrong timing (such as naturally activating tissue), or to not achieving the pacing, the determination engine supplements the pacing techniques by understanding how electrical signals flow through a circuit (e.g., how waves propagate). For instance, determines that a stimulating pacing signal is captured from at least a portion of an anatomical structure; determines a time duration between the stimulating pacing signal and a subsequent response from the portion of an anatomical structure; and outputs the time duration.

One or more advantages, technical effects, and/or benefits of the determination engine can include providing cardiac physicians and medical personnel, after a stimulation, an automatic determination of a PPI based on detecting a signal that is read after a last stimulation signal. Thus, the determination engine particularly utilizes and transforms medical device equipment to enable/implement automatic determinations of a PPI that are otherwise not currently available or currently performed by cardiac physicians and medical personnel (e.g., such that a user may not need to track a time between a last stimulation and a reading of a signal).

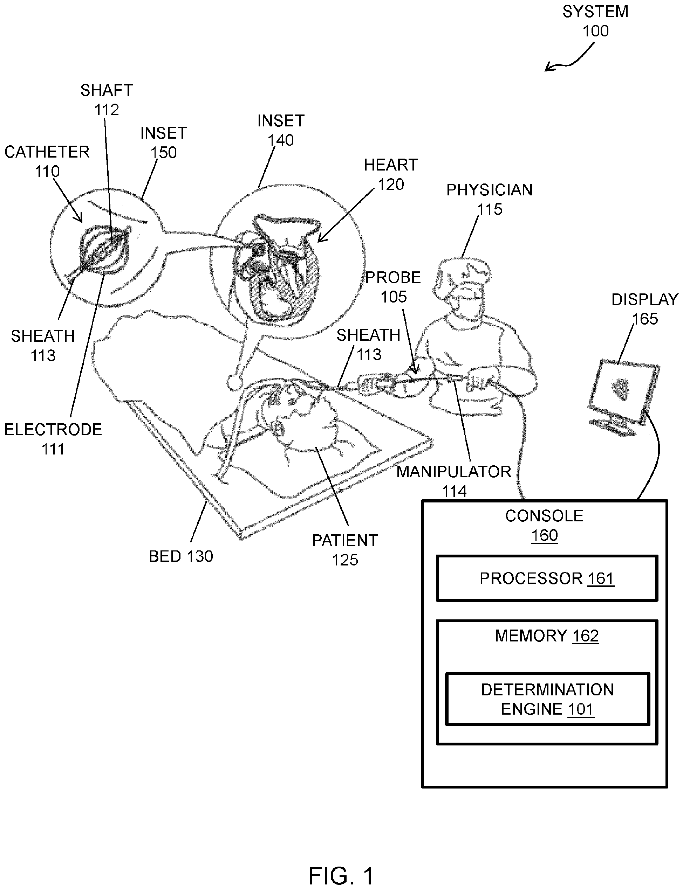

FIG. 1 is a diagram of an example system (e.g., medical device equipment), shown as a system 100, in which one or more features of the subject matter herein can be implemented according to one or more embodiments. All or part of the system 100 can be used to collect information (e.g., biometric data and/or a training dataset) and/or used to implement a machine learning and/or an artificial intelligence algorithm (e.g., a determination engine 101) as described herein. The system 100, as illustrated, includes a probe 105 with a catheter 110 (including at least one electrode 111), a shaft 112, a sheath 113, and a manipulator 114. The system 100, as illustrated, also includes a physician 115 (or a medical professional or clinician), a heart 120, a patient 125, and a bed 130 (or a table). Note that insets 140 and 150 show the heart 120 and the catheter 110 in greater detail. The system 100 also, as illustrated, includes a console 160 (including one or more processors 161 and memories 162) and a display 165. Note further each element and/or item of the system 100 is representative of one or more of that element and/or that item. The example of the system 100 shown in FIG. 1 can be modified to implement the embodiments disclosed herein. The disclosed embodiments can similarly be applied using other system components and settings. Additionally, the system 100 can include additional components, such as elements for sensing electrical activity, wired or wireless connectors, processing and display devices, or the like.

The system 100 can be utilized to detect, diagnose, and/or treat cardiac conditions (e.g., using the determination engine 101). Cardiac conditions, such as cardiac arrhythmias, persist as common and dangerous medical ailments, especially in the aging population. For instance, the system 100 can be part of a surgical system (e.g., CARTO^{®} system sold by Biosense Webster) that is configured to obtain biometric data (e.g., anatomical and electrical measurements of a patient's organ, such as the heart 120) and perform a cardiac ablation procedure. More particularly, treatments for cardiac conditions such as cardiac arrhythmia often require obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 120. Such locating may be done via an electrophysiological investigation during which electrical potentials are detected spatially resolved with a mapping catheter (e.g., the catheter 110) introduced into the chamber of the heart 120. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. In this case, the determination engine 101 can be directly stored and executed by the catheter 110.

In patients (e.g., the patient 125) with normal sinus rhythm (NSR), the heart (e.g., the heart 120), which includes atrial, ventricular, and excitatory conduction tissue, is electrically excited to beat in a synchronous, patterned fashion. Note that this electrical excitement can be detected as intracardiac electrocardiogram (IC ECG) data or the like.

In patients (e.g., the patient 125) with a cardiac arrhythmia (e.g., atrial fibrillation or aFib), abnormal regions of cardiac tissue do not follow a synchronous beating cycle associated with normally conductive tissue, which is in contrast to patients with NSR. Instead, the abnormal regions of cardiac tissue aberrantly conduct to adjacent tissue, thereby disrupting the cardiac cycle into an asynchronous cardiac rhythm. Note that this asynchronous cardiac rhythm can also be detected as the IC ECG data. Such abnormal conduction has been previously known to occur at various regions of the heart 120, for example, in the region of the sino-atrial (SA) node, along the conduction pathways of the atrioventricular (AV) node, or in the cardiac muscle tissue forming the walls of the ventricular and atrial cardiac chambers. There are other conditions, such as flutter, where the pattern of abnormally conducting tissues lead to reentry paths such that the chamber beats in a regular pattern that can be multiple times the sinus rhythm.

In support of the system 100 detecting, diagnosing, and/or treating cardiac conditions, the probe 105 can be navigated by the physician 115 into the heart 120 of the patient 125 lying on the bed 130. For instance, the physician 115 can insert the shaft 112 through the sheath 113, while manipulating a distal end of the shaft 112 using the manipulator 114 near the proximal end of the catheter 110 and/or deflection from the sheath 113. As shown in an inset 140, the catheter 110 can be fitted at the distal end of the shaft 112. The catheter 110 can be inserted through the sheath 113 in a collapsed state and can be then expanded within the heart 120.

Generally, electrical activity at a point in the heart 120 may be typically measured by advancing the catheter 110 containing an electrical sensor at or near its distal tip (e.g., the at least one electrode 111) to that point in the heart 120, contacting the tissue with the sensor and acquiring data at that point. One drawback with mapping a cardiac chamber using a catheter type containing only a single, distal tip electrode is the long period of time required to accumulate data on a point-by-point basis over the requisite number of points required for a detailed map of the chamber as a whole. Accordingly, multiple-electrode catheters (e.g., the catheter 110) have been developed to simultaneously measure electrical activity at multiple points in the heart chamber.

The catheter 110, which can include the at least one electrode 111 and a catheter needle coupled onto a body thereof, can be configured to obtain biometric data, such as electrical signals of an intra-body organ (e.g., the heart 120), and/or to ablate tissue areas of thereof (e.g., a cardiac chamber of the heart 120). Note that the electrodes 111 are representative of any like elements, such as tracking coils, piezoelectric transducer, electrodes, or combination of elements configured to ablate the tissue areas or to obtain the biometric data. According to one or more embodiments, the catheter 110 can include one or more position sensors that used are to determine trajectory information. The trajectory information can be used to infer motion characteristics, such as the contractility of the tissue.

Biometric data (e.g., patient biometrics, patient data, or patient biometric data) can include one or more of local activation times (LATs), electrical activity, topology, bipolar mapping, reference activity, ventricle activity, dominant frequency, impedance, or the like. The LAT can be a point in time of a threshold activity corresponding to a local activation, calculated based on a normalized initial starting point. Electrical activity can be any applicable electrical signals that can be measured based on one or more thresholds and can be sensed and/or augmented based on signal to noise ratios and/or other filters. A topology can correspond to the physical structure of a body part or a portion of a body part and can correspond to changes in the physical structure relative to different parts of the body part or relative to different body parts. A dominant frequency can be a frequency or a range of frequency that is prevalent at a portion of a body part and can be different in different portions of the same body part. For example, the dominant frequency of a PV of a heart can be different than the dominant frequency of the right atrium of the same heart. Impedance can be the resistance measurement at a given area of a body part.

Examples of biometric data include, but are not limited to, patient identification data, IC ECG data, bipolar intracardiac reference signals, anatomical and electrical measurements, trajectory information, body surface (BS) ECG data, historical data, brain biometrics, blood pressure data, ultrasound signals, radio signals, audio signals, a two- or three-dimensional image data, blood glucose data, and temperature data. The biometrics data can be used, generally, to monitor, diagnosis, and treatment any number of various diseases, such as cardiovascular diseases (e.g., arrhythmias, cardiomyopathy, and coronary artery disease) and autoimmune diseases (e.g., type I and type II diabetes). Note that BS ECG data can include data and signals collected from electrodes on a surface of a patient, IC ECG data can include data and signals collected from electrodes within the patient, and ablation data can include data and signals collected from tissue that has been ablated. Further, BS ECG data, IC ECG data, and ablation data, along with catheter electrode position data, can be derived from one or more procedure recordings.

For example, the catheter 110 can use the electrodes 111 to implement intravascular ultrasound and/or MRI catheterization to image the heart 120 (e.g., obtain and process the biometric data). Inset 150 shows the catheter 110 in an enlarged view, inside a cardiac chamber of the heart 120. Although the catheter 110 is shown to be a point catheter, it will be understood that any shape that includes one or more electrodes 111 can be used to implement the embodiments disclosed herein.

Examples of the catheter 110 include, but are not limited to, a linear catheter with multiple electrodes, a balloon catheter including electrodes dispersed on multiple spines that shape the balloon, a lasso or loop catheter with multiple electrodes, or any other applicable shape. Linear catheters can be fully or partially elastic such that it can twist, bend, and or otherwise change its shape based on received signal and/or based on application of an external force (e.g., cardiac tissue) on the linear catheter. The balloon catheter can be designed such that when deployed into a patient's body, its electrodes can be held in intimate contact against an endocardial surface. As an example, a balloon catheter can be inserted into a lumen, such as a pulmonary vein (PV). The balloon catheter can be inserted into the PV in a deflated state, such that the balloon catheter does not occupy its maximum volume while being inserted into the PV. The balloon catheter can expand while inside the PV, such that those electrodes on the balloon catheter are in contact with an entire circular section of the PV. Such contact with an entire circular section of the PV, or any other lumen, can enable efficient imaging and/or ablation.

According to other examples, body patches and/or BS electrodes may also be positioned on or proximate to a body of the patient 125. The catheter 110 with the one or more electrodes 111 can be positioned within the body (e.g., within the heart 120) and a position of the catheter 110 can be determined by the 100 system based on signals transmitted and received between the one or more electrodes 111 of the catheter 110 and the body patches and/or BS electrodes. Additionally, the electrodes 111 can sense the biometric data from within the body of the patient 125, such as within the heart 120 (e.g., the electrodes 111 sense the electrical potential of the tissue in real time). The biometric data can be associated with the determined position of the catheter 110 such that a rendering of the patient's body part (e.g., the heart 120) can be displayed and show the biometric data overlaid on a shape of the body part.

The probe 105 and other items of the system 100 can be connected to the console 160. The console 160 can include any computing device, which employs the machine learning and/or an artificial intelligence algorithm (represented as the determination engine 101). According to an embodiment, the console 160 includes the one or more processors 161 (any computing hardware) and the memory 162 (any non-transitory tangible media), where the one or more processors 161 execute computer instructions with respect the determination engine 101 and the memory 162 stores these instructions for execution by the one or more processors 161. For instance, the console 160 can be configured to receive and process the biometric data and determine if a given tissue area conducts electricity. In some embodiments, the console 160 can be further programmed by the determination engine 101 (in software) to carry out the functions of determining that a stimulating pacing signal is captured from at least a portion of an anatomical structure, determining a time duration between the stimulating pacing signal and a subsequent response from the portion of an anatomical structure, and outputting the time duration. According to one or more embodiments, the determination engine 101 can be external to the console 160 and can be located, for example, in the catheter 110, in an external device, in a mobile device, in a cloud-based device, or can be a standalone processor. In this regard, the determination engine 101 can be transferable/downloaded in electronic form, over a network.

In an example, the console 160 can be any computing device, as noted herein, including software (e.g., the determination engine 101) and/or hardware (e.g., the processor 161 and the memory 162), such as a general-purpose computer, with suitable front end and interface circuits for transmitting and receiving signals to and from the probe 105, as well as for controlling the other components of the system 100. For example, the front end and interface circuits include input/output (I/O) communication interfaces that enables the console 160 to receive signals from and/or transfer signals to the at least one electrode 111. The console 160 can include real-time noise reduction circuitry typically configured as a field programmable gate array (FPGA), followed by an analog-to-digital (A/D) ECG or electrocardiograph/electromyogram (EMG) signal conversion integrated circuit. The console 160 can pass the signal from an A/D ECG or EMG circuit to another processor and/or can be programmed to perform one or more functions disclosed herein.

The display 165, which can be any electronic device for the visual presentation of the biometric data, is connected to the console 160. According to an embodiment, during a procedure, the console 160 can facilitate on the display 165 a presentation of a body part rendering to the physician 115 and store data representing the body part rendering in the memory 162. For instance, maps depicting motion characteristics can be rendered/constructed based on the trajectory information sampled at a sufficient number of points in the heart 120. As an example, the display 165 can include a touchscreen that can be configured to accept inputs from the medical professional 115, in addition to presenting the body part rendering.

In some embodiments, the physician 115 can manipulate the elements of the system 100 and/or the body part rendering using one or more input devices, such as a touch pad, a mouse, a keyboard, a gesture recognition apparatus, or the like. For example, an input device can be used to change a position of the catheter 110, such that rendering is updated. Note that the display 165 can be located at a same location or a remote location, such as a separate hospital or in separate healthcare provider networks.

According to one or more embodiments, the system 100 can also obtain the biometric data using ultrasound, computed tomography (CT), MRI, or other medical imaging techniques utilizing the catheter 110 or other medical equipment. For instance, the system 100 can obtain ECG data and/or anatomical and electrical measurements of the heart 120 (e.g., the biometric data) using one or more catheters 110 or other sensors. More particularly, the console 160 can be connected, by a cable, to BS electrodes, which include adhesive skin patches affixed to the patient 125. The BS electrodes can procure/generate the biometric data in the form of the BS ECG data. For instance, the processor 161 can determine position coordinates of the catheter 110 inside the body part (e.g., the heart 120) of the patient 125. The position coordinates may be based on impedances or electromagnetic fields measured between the BS electrodes and the electrode 111 of the catheter 110 or other electromagnetic components. Additionally, or alternatively, location pads, which generate magnetic fields used for navigation, may be located on a surface of the bed 130 and may be separate from the bed 130. The biometric data can be transmitted to the console 160 and stored in the memory 162. Alternatively, or in addition, the biometric data may be transmitted to a server, which may be local or remote, using a network as further described herein.

According to one or more embodiments, the catheter 110 may be configured to ablate tissue areas of a cardiac chamber of the heart 120. Inset 150 shows the catheter 110 in an enlarged view, inside a cardiac chamber of the heart 120. For instance, ablation electrodes, such as the at least one electrode 111, may be configured to provide energy to tissue areas of an intra-body organ (e.g., the heart 120). The energy may be thermal energy and may cause damage to the tissue area starting from the surface of the tissue area and extending into the thickness of the tissue area. The biometric data with respect to ablation procedures (e.g., ablation tissues, ablation locations, etc.) can be considered ablation data.

According to an example, with respect to obtaining the biometric data, a multi-electrode catheter (e.g., the catheter 110) can be advanced into a chamber of the heart 120. Anteroposterior (AP) and lateral fluorograms can be obtained to establish the position and orientation of each of the electrodes. ECGs can be recorded from each of the electrodes 111 in contact with a cardiac surface relative to a temporal reference, such as the onset of the P-wave in sinus rhythm from a BS ECG and/or signals from electrodes 111 of the catheter 110 placed in a coronary sinus (CS), which can be referred to as a CS catheter (CSC). The system, as further disclosed herein, may differentiate between those electrodes that register electrical activity and those that do not due to absence of close proximity to the endocardial wall. After initial ECGs are recorded, the catheter may be repositioned, and fluorograms and ECGs may be recorded again. An electrical map (e.g., via cardiac mapping) can then be constructed from iterations of the process above.

Cardiac mapping can be implemented using one or more techniques. Generally, mapping of cardiac areas such as cardiac regions, tissue, veins, arteries and/or electrical pathways of the heart 120 may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping (which is an example of heart imaging) may include mapping based on one or more modalities such as, but not limited to LAT, local activation velocity, an electrical activity, a topology, a bipolar mapping, a dominant frequency, or an impedance. Data (e.g., biometric data) corresponding to multiple modalities may be captured using a catheter (e.g., the catheter 110) inserted into a patient's body and may be provided for rendering at the same time or at different times based on corresponding settings and/or preferences of the physician 115.

As an example of a first technique, cardiac mapping may be implemented by sensing an electrical property of heart tissue, for example, LAT, as a function of the precise location within the heart 120. The corresponding data (e.g., biometric data) may be acquired with one or more catheters (e.g., the catheter 110) that are advanced into the heart 1120 and that have electrical and location sensors (e.g., the electrodes 111) in their distal tips. As specific examples, location and electrical activity may be initially measured on about 10 to about 20 points on the interior surface of the heart 120. These data points may be generally sufficient to generate a preliminary reconstruction or map of the cardiac surface to a satisfactory quality. The preliminary map may be combined with data taken at additional points to generate a more comprehensive map of the heart's electrical activity. In clinical settings, it is not uncommon to accumulate data at 100 or more sites (e.g., several thousand) to generate a detailed, comprehensive map of heart chamber electrical activity. The generated detailed map may then serve as the basis for deciding on a therapeutic course of action, for example, tissue ablation as described herein, to alter the propagation of the heart's electrical activity and to restore normal heart rhythm.

Further, cardiac mapping can be generated based on detection of intracardiac electrical potential fields (e.g., which is an example of IC ECG data and/or bipolar intracardiac reference signals). A non-contact technique to simultaneously acquire a large amount of cardiac electrical information may be implemented. For example, a catheter type having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes). As another more specific example, the catheter may include other multispline catheters, such as five soft flexible branches, eight radial splines, or a parallel splined pancake turner type (e.g., any of which may have a total of 42 electrodes).

As example of electrical or cardiac mapping, an electrophysiological cardiac mapping system and technique based on a non-contact and non-expanded multi-electrode catheter (e.g., the catheter 110) can be implemented. ECGs may be obtained with one or more catheters 110 having multiple electrodes (e.g., such as between 42 to 122 electrodes). According to this implementation, knowledge of the relative geometry of the probe and the endocardium can be obtained by an independent imaging modality, such as transesophageal echocardiography. After the independent imaging, non-contact electrodes may be used to measure cardiac surface potentials and construct maps therefrom (e.g., in some cases using bipolar intracardiac reference signals). This technique can include the following steps (after the independent imaging step): (a) measuring electrical potentials with a plurality of electrodes disposed on a probe positioned in the heart 120; (b) determining the geometric relationship of the probe surface and the endocardial surface and/or other reference; (c) generating a matrix of coefficients representing the geometric relationship of the probe surface and the endocardial surface; and (d) determining endocardial potentials based on the electrode potentials and the matrix of coefficients.

As another example of electrical or cardiac mapping, a technique and apparatus for mapping the electrical potential distribution of a heart chamber can be implemented. An intra-cardiac multi-electrode mapping catheter assembly can be inserted into the heart 120. The mapping catheter (e.g., the catheter 110) assembly can include a multi-electrode array with one or more integral reference electrodes (e.g., one or the electrodes 111) or a companion reference catheter.

According to one or more embodiments, the electrodes may be deployed in the form of a substantially spherical array, which may be spatially referenced to a point on the endocardial surface by the reference electrode or by the reference catheter this is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

In view of electrical or cardiac mapping and according to another example, the catheter 110 can be a heart mapping catheter assembly that may include an electrode array defining a number of electrode sites. The heart mapping catheter assembly can also include a lumen to accept a reference catheter having a distal tip electrode assembly that may be used to probe the heart wall. The map heart mapping catheter assembly can include a braid of insulated wires (e.g., having 24 to 64 wires in the braid), and each of the wires may be used to form electrode sites. The heart mapping catheter assembly may be readily positioned in the heart 120 to be used to acquire electrical activity information from a first set of non-contact electrode sites and/or a second set of in-contact electrode sites.

Further, according to another example, the catheter 110 that can implement mapping electrophysiological activity within the heart can include a distal tip that is adapted for delivery of a stimulating pulse for pacing the heart or an ablative electrode for ablating tissue in contact with the tip. This catheter 110 can further include at least one pair of orthogonal electrodes to generate a difference signal indicative of the local cardiac electrical activity adjacent the orthogonal electrodes.

As noted herein, the system 100 can be utilized to detect, diagnose, and/or treat cardiac conditions. In example operation, a process for measuring electrophysiologic data in a heart chamber may be implemented by the system 100. The process may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In preferred embodiments, the array is said to have from 60 to 64 electrodes.

As another example operation, cardiac mapping may be implemented by the system 100 using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of the probe 105 (e.g., a treatment catheter shown as catheter 110) at the later time may be displayed and the probe 105 may be overlaid onto the one or more ultrasound slices.

In view of the system 100, it is noted that cardiac arrhythmias, including atrial arrhythmias, may be of a multiwavelet reentrant type, characterized by multiple asynchronous loops of electrical impulses that are scattered about the atrial chamber and are often self-propagating (e.g., another example of the IC ECG data). Alternatively, or in addition to the multiwavelet reentrant type, cardiac arrhythmias may also have a focal origin, such as when an isolated region of tissue in an atrium fires autonomously in a rapid, repetitive fashion (e.g., another example of the IC ECG data). Ventricular tachycardia (V-tach or VT) is a tachycardia, or fast heart rhythm that originates in one of the ventricles of the heart (e.g., Tachycardia can be a heart rate over 100 beats per minute). This is a potentially life-threatening arrhythmia because it may lead to ventricular fibrillation and sudden death.

For example, aFib occurs when the normal electrical impulses (e.g., another example of the IC ECG data) generated by the sinoatrial node are overwhelmed by disorganized electrical impulses (e.g., signal interference) that originate in the atria veins and PVs causing irregular impulses to be conducted to the ventricles. An irregular heartbeat results and may last from minutes to weeks, or even years. aFib is often a chronic condition that leads to a small increase in the risk of death often due to strokes. A line of treatment for aFib is medication that either slows the heart rate or revert the heart rhythm back to normal. Additionally, persons with aFib are often given anticoagulants to protect them from the risk of stroke. The use of such anticoagulants comes with its own risk of internal bleeding. In some patients, medication is not sufficient and their aFib is deemed to be drug-refractory, i.e., untreatable with standard pharmacological interventions. Synchronized electrical cardioversion may also be used to convert aFib to a normal heart rhythm. Alternatively, aFib patients are treated by catheter ablation.

A catheter ablation-based treatment may include mapping the electrical properties of heart tissue, especially the endocardium and the heart volume, and selectively ablating cardiac tissue by application of energy. Electrical or cardiac mapping (e.g., implemented by any electrophysiological cardiac mapping system and technique described herein) includes creating a map of electrical potentials (e.g., a voltage map) of the wave propagation along the heart tissue or a map of arrival times (e.g., a LAT map) to various tissue located points. Electrical or cardiac mapping (e.g., a cardiac map) may be used for detecting local heart tissue dysfunction. Ablations, such as those based on cardiac mapping, can cease or modify the propagation of unwanted electrical signals from one portion of the heart 120 to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. Another example of an energy delivery technique includes irreversible electroporation (IRE), which provides high electric fields that damage cell membranes. In a two-step procedure (e.g., mapping followed by ablation) electrical activity at points within the heart 120 is typically sensed and measured by advancing the catheter 110 containing one or more electrical sensors (e.g., electrodes 111) into the heart 120 and obtaining/acquiring data at a multiplicity of points (e.g., as biometric data generally, or as ECG data specifically). This ECG data is then utilized to select the endocardial target areas, at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems to reconstruct the anatomy of the heart chamber of interest. In this regard, the determination engine 101 employed by the system 100 herein manipulates and evaluates the biometric data generally, or the ECG data specifically, to produce improved tissue data that enables more accurate diagnosis, images, scans, and/or maps for treating an abnormal heartbeat or arrhythmia. For example, cardiologists rely upon software, such as the Complex Fractionated Atrial Electrograms (CFAE) module of the CARTO^{®} 3 3D mapping system, produced by Biosense Webster, Inc. (Diamond Bar, Calif.), to generate and analyze ECG data. The determination engine 101 of the system 100 enhances this software to generate and analyze the improved biometric data, which further provide multiple pieces of information regarding electrophysiological properties of the heart 120 (including the scar tissue) that represent cardiac substrates (anatomical and functional) of aFib.

Accordingly, the system 100 can implement a 3D mapping system, such as CARTO^{®} 3 3D mapping system, to localize the potential arrhythmogenic substrate of the cardiomyopathy in terms of abnormal ECG detection. The substrate linked to these cardiac conditions is related to the presence of fragmented and prolonged ECGs in the endocardial and/or epicardial layers of the ventricular chambers (right and left). In general, abnormal tissue is characterized by low-voltage ECGs. However, initial clinical experience in endo-epicardial mapping indicates that areas of low-voltage are not always present as the sole arrhythmogenic mechanism in such patients. In fact, areas of low or medium voltage may exhibit ECG fragmentation and prolonged activities during sinus rhythm, which corresponds to the critical isthmus identified during sustained and organized ventricular arrhythmias, e.g., applies only to non-tolerated ventricular tachycardias. Moreover, in many cases, ECG fragmentation and prolonged activities are observed in the regions showing a normal or near-normal voltage amplitude (>1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

As another example operation, cardiac mapping may be implemented by the system 100 using one or more multiple-electrode catheters (e.g., the catheter 110). Multiple-electrode catheters are used to stimulate and map electrical activity in the heart 120 and to ablate sites of aberrant electrical activity. In use, the multiple-electrode catheter is inserted into a major vein or artery, e.g., femoral vein, and then guided into the chamber of the heart 120 of concern. A typical ablation procedure involves the insertion of the catheter 110 having at least one electrode 111 at its distal end, into a heart chamber. A reference electrode is provided, taped to the skin of the patient or by means of a second catheter that is positioned in or near the heart or selected from one or the other electrodes 111 of the catheter 110. Radio frequency (RF) current is applied to a tip electrode 111 of the ablating catheter 110, and current flows through the media that surrounds it (e.g., blood and tissue) toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the tip electrode 111 also occurs as a result of conduction from the heated tissue to the electrode itself. If the electrode temperature becomes sufficiently high, possibly above 60 degrees Celsius, a thin transparent coating of dehydrated blood protein can form on the surface of the electrode 111. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter 110 must be removed from the body and the tip electrode 111 cleaned.

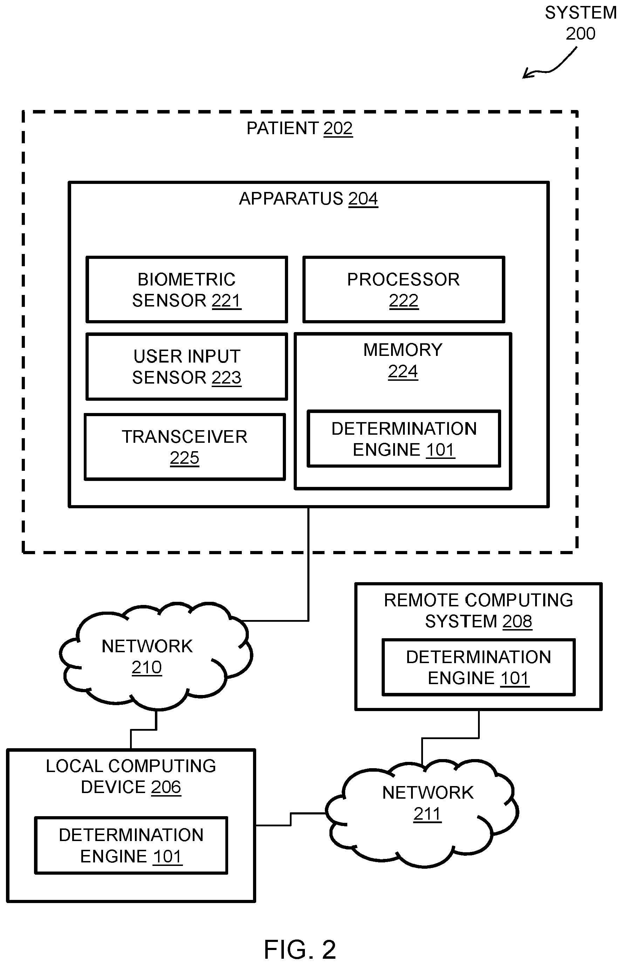

Turning now to FIG. 2, a diagram of a system 200 in which one or more features of the disclosure subject matter can be implemented is illustrated according to one or more embodiments. The system 200 includes, in relation to a patient 202 (e.g., an example of the patient 125 of FIG. 1), an apparatus 204, a local computing device 206, a remote computing system 208, a first network 210, and a second network 211. Further, the apparatus 204 can include a biometric sensor 221 (e.g., an example of the catheter 110 of FIG. 1), a processor 222, a user input (UI) sensor 223, a memory 224, and a transceiver 225. Note that the determination engine 101 of FIG. 1 is reused in FIG. 2 for ease of explanation and brevity.

According to an embodiment, the apparatus 204 can be an example of the system 100 of FIG. 1, where the apparatus 204 can include both components that are internal to the patient and components that are external to the patient. According to an embodiment, the apparatus 204 can be an apparatus that is external to the patient 202 that includes an attachable patch (e.g., that attaches to a patient's skin). According to another embodiment, the apparatus 204 can be internal to a body of the patient 202 (e.g., subcutaneously implantable), where the apparatus 204 can be inserted into the patient 202 via any applicable manner including orally injecting, surgical insertion via a vein or artery, an endoscopic procedure, or a laparoscopic procedure. According to an embodiment, while a single apparatus 204 is shown in FIG. 2, example systems may include a plurality of apparatuses.

Accordingly, the apparatus 204, the local computing device 206, and/or the remote computing system 208 can be programed to execute computer instructions with respect the determination engine 101. As an example, the memory 223 stores these instructions for execution by the processor 222 so that the apparatus 204 can receive and process the biometric data via the biometric sensor 201. In this way, the processor 222 and the memory 223 are representative of processors and memories of the local computing device 206 and/or the remote computing system 208.

The apparatus 204, local computing device 206, and/or the remote computing system 208 can be any combination of software and/or hardware that individually or collectively store, execute, and implement the determination engine 101 and functions thereof. Further, the apparatus 204, local computing device 206, and/or the remote computing system 208 can be an electronic, computer framework comprising and/or employing any number and combination of computing device and networks utilizing various communication technologies, as described herein. The apparatus 204, local computing device 206, and/or the remote computing system 208 can be easily scalable, extensible, and modular, with the ability to change to different services or reconfigure some features independently of others.

The networks 210 and 211 can be a wired network, a wireless network, or include one or more wired and wireless networks. According to an embodiment, the network 210 is an example of a short-range network (e.g., local area network (LAN), or personal area network (PAN)). Information can be sent, via the network 210, between the apparatus 204 and the local computing device 206 using any one of various short-range wireless communication protocols, such as Bluetooth, Wi-Fi, Zigbee, Z-Wave, near field communications (NFC), ultra-band, Zigbee, or infrared (IR). Further, the network 211 is an example of one or more of an Intranet, a local area network (LAN), a wide area network (WAN), a metropolitan area network (MAN), a direct connection or series of connections, a cellular telephone network, or any other network or medium capable of facilitating communication between the local computing device 206 and the remote computing system 208. Information can be sent, via the network 211, using any one of various long-range wireless communication protocols (e.g., TCP/IP, HTTP, 3G, 4G/LTE, or 5G/New Radio). Note that for either network 210 and 211 wired connections can be implemented using Ethernet, Universal Serial Bus (USB), RJ-11 or any other wired connection and wireless connections can be implemented using Wi-Fi, WiMAX, and Bluetooth, infrared, cellular networks, satellite or any other wireless connection methodology.

In operation, the apparatus 204 can continually or periodically obtain, monitor, store, process, and communicate via network 210 the biometric data associated with the patient 202. Further, the apparatus 204, local computing device 206, and/ the remote computing system 208 are in communication through the networks 210 and 211 (e.g., the local computing device 206 can be configured as a gateway between the apparatus 204 and the remote computing system 208). For instance, the apparatus 204 can be an example of the system 100 of FIG. 1 configured to communicate with the local computing device 206 via the network 210. The local computing device 206 can be, for example, a stationary/standalone device, a base station, a desktop/laptop computer, a smart phone, a smartwatch, a tablet, or other device configured to communicate with other devices via networks 211 and 210. The remote computing system 208, implemented as a physical server on or connected to the network 211 or as a virtual server in a public cloud computing provider (e.g., Amazon Web Services (AWS) ^{®}) of the network 211, can be configured to communicate with the local computing device 206 via the network 211. Thus, the biometric data associated with the patient 202 can be communicated throughout the system 200.

Elements of the apparatus 204 are now described. The biometric sensor 221 may include, for example, one or more transducers configured to convert one or more environmental conditions into an electrical signal, such that different types of biometric data are observed/obtained/acquired. For example, the biometric sensor 221 can include one or more of an electrode (e.g., the electrode 111 of FIG. 1), a temperature sensor (e.g., thermocouple), a blood pressure sensor, a blood glucose sensor, a blood oxygen sensor, a pH sensor, an accelerometer, and a microphone.

The processor 222, in executing the determination engine 101, can be configured to receive, process, and manage the biometric data acquired by the biometric sensor 221, and communicate the biometric data to the memory 224 for storage and/or across the network 210 via the transceiver 225. Biometric data from one or more other apparatuses 204 can also be received by the processor 222 through the transceiver 225. Also, as described in more detail herein, the processor 222 may be configured to respond selectively to different tapping patterns (e.g., a single tap or a double tap) received from the UI sensor 223, such that different tasks of a patch (e.g., acquisition, storing, or transmission of data) can be activated based on the detected pattern. In some embodiments, the processor 222 can generate audible feedback with respect to detecting a gesture.

The UI sensor 223 includes, for example, a piezoelectric sensor or a capacitive sensor configured to receive a user input, such as a tapping or touching. For example, the UI sensor 223 can be controlled to implement a capacitive coupling, in response to tapping or touching a surface of the apparatus 204 by the patient 202. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface, such that the tapping or touching of the surface activates the monitoring device.

The memory 224 is any non-transitory tangible media, such as magnetic, optical, or electronic memory (e.g., any suitable volatile and/or non-volatile memory, such as random-access memory or a hard disk drive). The memory 224 stores the computer instructions for execution by the processor 222.

The transceiver 225 may include a separate transmitter and a separate receiver. Alternatively, the transceiver 225 may include a transmitter and receiver integrated into a single device.

In operation, the apparatus 204, utilizing the determination engine 101, observes/obtains the biometric data of the patient 202 via the biometric sensor 221, stores the biometric data in the memory, and shares this biometric data across the system 200 via the transceiver 225. The determination engine 101 can then utilize models, neural networks, machine learning, and/or artificial intelligence to provide cardiac physicians and medical personnel, after a stimulation, an automatic determination of a PPI based on detecting a signal that is read after a last stimulation signal.

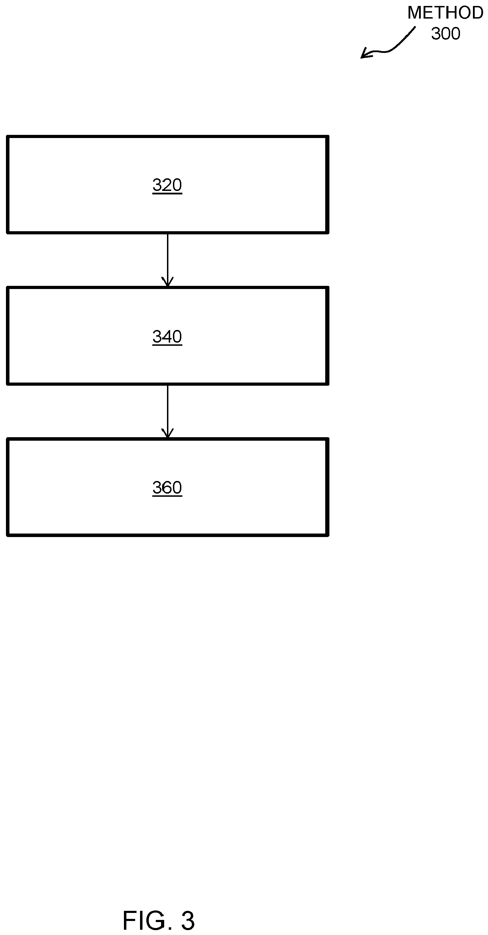

Turning now to FIG. 3, a method 300 (e.g., performed by the determination engine 101 of FIG. 1 and/or of FIG. 2) is illustrated according to one or more exemplary embodiments. The method 300 addresses a need to automatically track a time between a last stimulation and a reading of a signal by providing a multi-step manipulation of electrical signals that enables an improved understanding an electrophysiology with more precision.

The method begins at block 320, where the determination engine 101 determines that a stimulating pacing signal is captured from at least a portion of an anatomical structure. As described herein, the anatomical structure can be any anatomical structure, body part, organ, or portion thereof (e.g., such as the heart or a ventricle or atria of the heart) can be a target for pacing, mapping, and diagnosis. Further, the pacing can be implemented by one or more catheters 110 (e.g., multi-electrode mapping catheter and/or a CSC) in connection with body patches and/or BS electrodes.

The stimulating pacing signal includes at least one signal from the pacing using the electrodes 111 of the catheter 110 (e.g., electrical pacing signals). Note that, in general, the pacing can be a train of sequential signals (e.g., signals 1, 2, and 3). In this case, the system 100 looks at the end of the sequence to perform the method 300 to determine that the stimulating pacing signal is captured (and that a next natural event of activity is detected). Additionally, note that an interval of each sequential pacing signal can vary (e.g., the signal 1 has a C1 interval, the signal 2 has the C1 interval, and interval 3 has a C2 interval).

According from for the one or more, capturing the stimulating pacing signal can be performed by specialized hardware of the system 100 (e.g., a pacing mechanism that knows when pacing occurs) and/or by an advanced reference algorithm (ARA) of the determination engine 101 (e.g., which knows when pacing occurs). Note that, when there is a capture, the specialized hardware of the system 100 and/or the ARA of the determination engine 101 recognizes that control of the anatomical structure has been gained.

For instance, when pacing a heart, if an atria is properly captured then ventricle activity is detected according to the pacing (the ventricle can contract in accordance of the pacing) by the system 100 and/or the determination engine 101. Practically, the system 100 and/or the determination engine 101 determines that heart tissue is being influenced, heart activity is synchronized with the pacing, and the heart is captured, when a BS signal at 100 milliseconds is detected after a first pacing; another BS signal at a same 100 milliseconds is detected after a second pacing; and so forth. Yet, if the ventricle activity has is no relation to the pacing or there is no syncing, then the system 100 and/or the determination engine 101 recognizes that there is no influencing the tissue of the heat and no tissue capture (e.g., control of the heart has not been gained).

According to one or more embodiments, in the case of a CSC being stationed (e.g., stationary relative to the multi-electrode mapping catheter) within a CS, a refractory period can be used by the CSC to detect proper atria capture (e.g., as electrical signals conduct differently in the CS by the CSC than elsewhere in the heart by the multi-electrode mapping catheter). For example, if a Tachycardia revolves around a left atria, the CSC detects an order and a direction of the waves of the Tachycardia (as these electrical signals revolve), along with any corresponding morphology. In contrast, during pacing, the electrical pacing signals clear the CSC differently as the electrical pacing signals have a different order, direction, and morphology than the Tachycardia waves. Detecting this difference indicates to the CSC (and correspondingly the determination engine 101) that the left atria was properly effective and the heart was captured by the pacing.

According to one or more embodiments, a method for a capture detection can utilize a presence of one or more catheters 110 in the heart 120. For example, when a proper captured is effective, a sequence of an activation on the CS catheter can change (e.g., pending wavefront propagation in the heart 120 and pacing site location). In some cases, as morphology changes between pre-pacing conditions to pacing conditions, there changed are measured to assess whether capture was successful. Further, in some scenarios (e.g., as pacing in isolated PV from a multi electrode catheter), a "local capture" can be identified following successful a capture.

At block 340, the determination engine 101 determines a time duration between the stimulating pacing signal and a subsequent response from the portion of the anatomical structure. The subsequent response includes a receipt of an electric wave from a particular point that was influenced by the electrical pacing signals (e.g., a sinus rhythm from an excitable gap of a circuit). In this regard, the determination engine 101 measures time between a last electrical pacing signal that was sent to the heart tissue and a first activity wave from the heart tissue. The measurement of time yields the PPI.

At block 360, the determination engine 101 outputs the time duration. The determination engine 101 can output the time duration in a map context, such as by the display 165 providing a visual rendering of the anatomical structure to the physician 115. The determination engine 101 can output the time duration for storage in the memory 162.

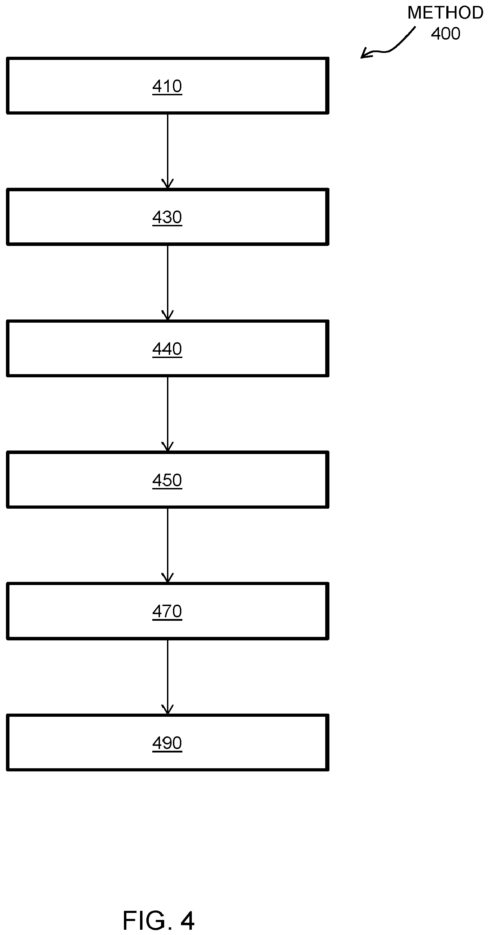

Turning now to FIG. 4, a method 400 (e.g., performed by the determination engine 101 of FIG. 1 and/or of FIG. 2) is illustrated according to one or more exemplary embodiments. The method 400 generally automatically determines a PPI based on first determining that a stimulated pacing signal triggered an area of an organ and then detecting subsequent automatic electrical activity after the last such stimulated pacing signal triggered the area of the organ. To determine whether the stimulated pacing signal triggered an area of an organ, one or more factors may be observed, including an understanding of the area of the organ (e.g., ventricle or atria).

The method begins at block 410, where the determination engine 101 provides a stimulating pacing signal to a portion of an anatomical structure (e.g., the determination engine 101 is pacing). The stimulating pacing signal can be provided at a first time (e.g., t0). In conjunction with the providing the stimulating pacing signal, the determination engine 101 can capture and/or detect additional information. The more additional information can include, but is not limited to, biometric data and/or a training dataset (e.g., one or more of LATs, electrical activity, topology, bipolar mapping, reference activity, ventricle activity, dominant frequency, impedance, or the like), along with pacing protocol parameters (e.g., pacing interval, sequence, and the like). In turn, the determination engine 101 collects a corpus of information at the first time to be used for machine learning and/or by artificial intelligence algorithm of the determination engine 101.

At block 430, the determination engine 101 captures the stimulating pacing signal. The stimulating pacing signal can be captured at a second time (e.g., t2 = t0+x1). In conjunction with the capturing the stimulating pacing signal, the determination engine 101 can capture and/or detect additional information (similar to block 410). Thus, the determination engine 101 adds to the corpus of information.

At block 440, the determination engine 101 captures a subsequent response from the portion of the anatomical structure. The stimulating pacing signal can be captured at a third time (e.g., t3 = t0+x3, where x3 > x2) or at the second time (e.g., t3 = t2). In conjunction with the capturing the subsequent response, the determination engine 101 can capture and/or detect additional information (similar to blocks 410 and 420). Thus, the determination engine 101 adds to the corpus of information.

At block 450, the determination engine 101 determines that the subsequent response was in connection/synchronized with the stimulating pacing signal (e.g., the anatomical structure was captured). For example, the subsequent response is a contraction of heart tissue in connection with the pacing. If the contraction is not connected, then the pacing did not capture the heart when providing the stimulated pacing signal. This determination can occur stimulating pacing signal can be captured at a fourth time (e.g., t4 = t0+x4, where x4 > x3) or at the third time (e.g., t3 = t4). The determination engine 101 can utilize the corpus of information to perform a big data analysis in making this determination. Note that the values of tO, x1, x2, x3, and/or x4 can be default values, user defined value, or machine determined values that are stored in the memory 162 and accessed by the determination engine 101 during the automatic operation of the method 400.

As another example, when providing the stimulated pacing signal in a ventricle, the determination engine 101 may detect a QRS complex or morphology (e.g., the subsequent response) on the body surface ECG. The QRS complex can be a combination of graphical deflections (e.g., three deflections) within an ECG and is usually a central main spike therein. The QRS complex can correspond to a depolarization of right and left ventricles of a heart and contraction of ventricular muscles.

As another example, when providing the stimulated pacing signal in an atria, the body surface ECG may not be sufficient to determine that the stimulated pacing signals triggered an area of an organ as even if there is a capture in the atria not all activities are translated to ventricle activity due to the natural design of the AV node of the heart and electric separation between the two chambers. Accordingly, a presence of activity in the CS and its timing/morphology relative to other beats may be used to determine that a stimulated pacing signal triggered an area of the atria, as described herein.

At block 470, the determination engine 101 determines a time duration between the stimulating pacing signal and the subsequent response. The determination of the time duration can be an automatic determination. Note that the time duration (and/or values associated therewith, such as values associated with a given location) are stored in the memory 162 and can be accessed by the determination engine 101 during subsequent automatic operations of the method 400.

At block 490, the determination engine 101 outputs the time duration. In an example, outputting the time duration can include a display of the time duration within the context of an electro-anatomical mapping, as described herein. Additionally, the outputting the time duration can include providing the time duration and the corpus of information to the remote computing system 208 for big data analysis. For example, the big data analysis can utilize multiple execution of the method 400, in conjunction with multiple manual PPI determinations, to predict and determine one or more of the first, second, third, and fourth times for executing the method. Additionally, the big data analysis can include measuring the validity of the captures and determination of the method 400 across multiple iterations thereof. In this way, the determination engine 101 can self-evaluate the automatic nature of captures and determinations therein to further increase an accuracy PPO observations

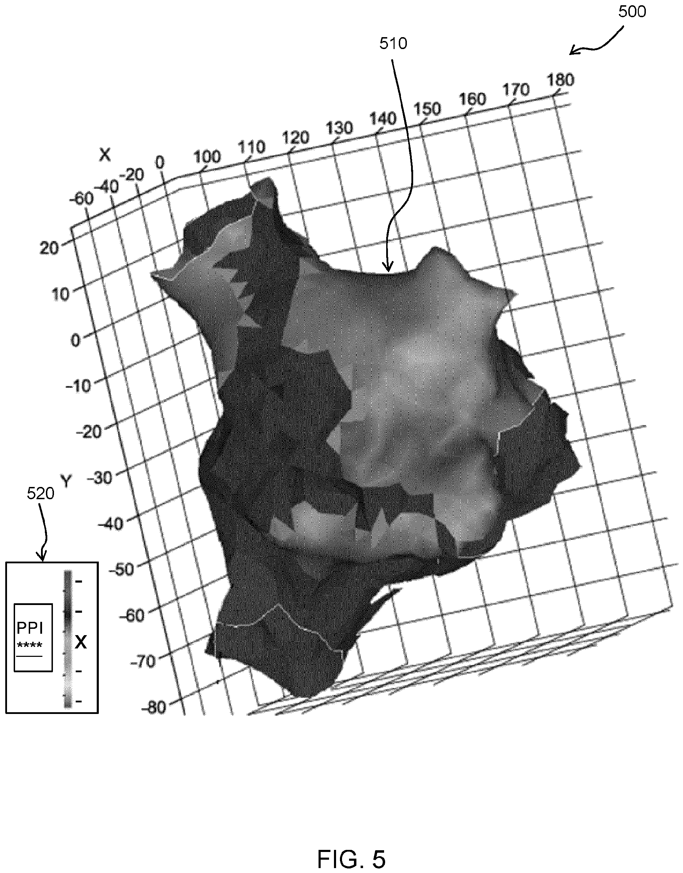

Turning to FIG. 5, an interface 500 is shown according to one or more embodiments. The graph 500 can be generated by the determination engine 101. The graph 500 can include a scan or mapping of an anatomical structure 510 accompanied by an interface 520 showing a time duration (e.g., a basic PPI measurement, where ** is representative of a number) and a color gradient that grades the time duration relevant to other PPIs and/or how far to the TCL.

The time duration can include an impediment value. The impediment value includes a value relative to a TCL, to prior found PPI, or to specific target PPI. In some cases, when the determination engine 101 performs PPI detection to find a circuit, the determination engine 101 can permit deviation from the TCL and the impediment value can be in relation to this deviation. The time duration can be a value for the PPI corresponding to a given location. Alternatively, the PPI corresponding to various given locations may be provided as part of a PPI map which provides respective PPIs for different locations of an organ. For example, the PPI for multiple locations on the portion of a heart may be displayed such that the PPI values are superimposed on a rendering of the portion of the heart. The PPI values may result in the determination of potential ablation sites, such as when used in conjunction with the electro-anatomical mapping and/or the big data analysis.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a terminal, base station, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

Aspects of the invention:
1. A method comprising:
   determining, by a determination engine stored as processor executable code on a memory, the memory being coupled to one or more processors executing the processor executable code, that a stimulating pacing signal is captured from at least a portion of an anatomical structure;
   determining, by the determination engine, a time duration between the stimulating pacing signal and a subsequent response from the portion of an anatomical structure; and
   outputting, by the determination engine, the time duration.
2. The method of aspect 1, wherein the determination engine provides the stimulating pacing signal to the portion of the anatomical structure.
3. The method of aspect 2, wherein the stimulating pacing signal is provided by a coronary sinus catheter stationed within the anatomical structure.
4. The method of aspect 1, wherein the time duration comprises an impediment value.
5. The method of aspect 1, wherein the determination engine outputs the time duration in a map context.
6. The method of aspect 1, wherein a pacing implemented by one or more catheters comprises the stimulating pacing signal.
7. The method of aspect 1, wherein the pacing comprises a train of sequential signals with the stimulating pacing signal being at an end of the train.
8. The method of aspect 7, wherein the subsequent response from the portion of the anatomical structure comprises a next natural event of activity.
9. The method of aspect 1, wherein the anatomical structure comprises a heart.
10. The method of aspect 1, wherein the determination engine utilizes the time duration and a corpus of information in a big data analysis to determine an accuracy of the time duration

## Claims

1. A system comprising:
a memory storing processor executable code for a determination engine; and
one or more processors configured to execute the processor executable code to cause the system to perform:
determining, by the determination engine, that a stimulating pacing signal is captured from at least a portion of an anatomical structure;
determining, by the determination engine, a time duration between the stimulating pacing signal and a subsequent response from the portion of an anatomical structure; and
outputting, by the determination engine, the time duration.

2. The system of claim 1, wherein the determination engine provides the stimulating pacing signal to the portion of the anatomical structure.

3. The system of claim 2, wherein the stimulating pacing signal is provided by a coronary sinus catheter stationed within the anatomical structure.

4. The system of claim 1, wherein the time duration comprises an impediment value.

5. The system of claim 1, wherein the determination engine outputs the time duration in a map context.

6. The system of claim 1, wherein a pacing implemented by one or more catheters comprises the stimulating pacing signal.

7. The system of claim 1, wherein the pacing comprises a train of sequential signals with the stimulating pacing signal being at an end of the train.

8. The system of claim 7, wherein the subsequent response from the portion of the anatomical structure comprises a next natural event of activity.

9. The system of claim 1, wherein the anatomical structure comprises a heart.

10. The system of claim 1, wherein the determination engine utilizes the time duration and a corpus of information in a big data analysis to determine an accuracy of the time duration.
